(19) Europäisches Patentamt
European Patent Office
Office européen des brevets

(11) Publication number: **0 393 789**
**A1**

(12) **EUROPEAN PATENT APPLICATION**

(21) Application number: **90200960.4**

(51) Int. Cl.5: **C07C 269/04, C07C 271/30**

(22) Date of filing: **18.04.90**

(30) Priority: **19.04.89 GB 8908870**

(43) Date of publication of application:
**24.10.90 Bulletin 90/43**

(84) Designated Contracting States:
**BE CH DE DK FR GB IT LI NL SE**

(71) Applicant: **SHELL INTERNATIONALE RESEARCH MAATSCHAPPIJ B.V.**
**Carel van Bylandtlaan 30**
**NL-2596 HR Den Haag(NL)**

(72) Inventor: **Goodall, Brian Leslie**
**3959 Clover Hill Road**
**Akron, Ohio 44313(US)**
Inventor: **Terlouw, Willem**
**Badhuisweg 3**
**NL-1031 CM Amsterdam(NL)**
Inventor: **Wullink-Schelvis, Annette Maria**
**Badhuisweg 3**
**NL-1031 CM Amsterdam(NL)**

(74) Representative: **Aalbers, Onno et al**
**P.O. Box 302**
**NL-2501 CH The Hague(NL)**

(54) **A process for the preparation of carbamates.**

(57) A process for preparing carbamates having the general formula I:

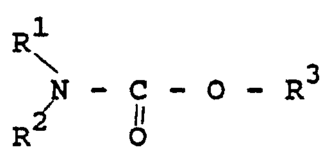

(I)

wherein $R^1$ represents a hydrogen atom or an alkyl group, $R^2$ represents an optionally substituted aryl group and $R^3$ represents an alkyl, haloalkyl, alkenyl, haloalkenyl, alkynyl, haloalkynyl group or an optionally substituted aryl or heteroaryl group, comprises reacting an amine having the general formula II:

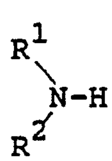

(II)

wherein $R^1$ and $R^2$ are as hereinbefore defined, or a symmetrical urea having the general formula III:

EP 0 393 789 A1

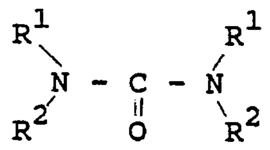 (III)

wherein $R^1$ and $R^2$ are as hereinbefore defined, carbon monoxide and oxygen in the presence of a catalyst comprising:

a) palladium;

b) an organo-nitrogen or organo-phosphorous ligand having a lone pair of electrons; and

c) a metal salt comprising a cation of a metal selected from the group consisting of copper, iron, vanadium, chromium, zinc, tin, uranium and cerium, and an anion of an acid having a pK of less than 2, except of a hydrohalogenic acid.

# A PROCESS FOR THE PREPARATION OF CARBAMATES

The present invention relates to a process for the preparation of carbamates and to carbamates prepared by means of such a process.

Carbamates are a known class of commercially available compounds having a wide range of uses. For example, certain members of this class of compounds exhibit activity as herbicides or insecticides and find appropriate use in the control of certain pests. In addition, carbamates can be used as precursors in a route to the preparation of isocyanates which, in turn, can be used to prepare a number of valuable compounds. In particular, isocyanates may be used to prepare acyl ureas, which themselves find use, for example, as insecticides.

European Patent application publication No. 0 036 895 (EP-A-0 036 895) discloses a process for preparing urethanes (alternatively named carbamates) which comprises reacting an aromatic amine with a hydroxyl compound, carbon monoxide and an oxidising agent, especially oxygen or a nitro compound, in the presence of a catalyst comprising either a noble metal and/or a noble metal salt, or a selenium-and/or sulphur-containing catalyst together with an additional basic compound as co-catalyst. Noble metals specifically listed in the specification of EP-A-0 036 895 include palladium, platinum, rhodium, ruthenium, osmium and iridium, whilst specific salts listed include oxides, sulphates, nitrates, halides and acetates. The catalyst is disclosed as preferably comprising, as a co-catalyst, a compound of tin, titanium, iron, mercury, nickel, vanadium, antimony, manganese, cobalt and/or copper. A list of possible compounds for use as co-catalysts given in the specification of EP-A-0 036 895 includes acetates, oxides, nitrates, phosphates, sulphates, tartrates, fluorides, molybdates, bromides, chlorides, iodides and oxalates. However, the only noble metal catalyst and co-catalyst combination specifically described and exemplified in EP-A-0-036 895 is one consisting of palladium chloride and ferric chloride.

Japanese patent application publication No. 60-139 659 (JP-A-60-139 659) discloses a process for preparing urethanes (carbamates) in which a primary or secondary amine is caused to react with carbon monoxide and an organic hydroxyl compound in the presence of an oxidising agent and a catalyst comprising a platinum-group metal or platinum-group metal compound, at least one halogen compound and at least one chelating reagent. A number of drawbacks of using a catalyst comprising a halogen compound are discussed in the specification of JP-A-60-139 659. For example, chlorides are said to be responsible for a large amount of corrosion in reaction vessels and pipework and are difficult to remove from the products of the reaction. However, the specification discloses that the halogen compounds play an important role in the reaction by acting as promoters. Therefore, in spite of the drawbacks associated with the use of halogen compounds, the processes disclosed and exemplified in JP-A-60-139 654 all rely on the use of a catalyst comprising a halogen compound as a promoter or co-catalyst. Platinum-group metals listed in the specification of JP-A-60-139 659 as being for use in the catalyst are palladium, rhodium, platinum, ruthenium, iridium and osmium, with platinum and rhodium being especially preferred. Halogen compounds may be either organic or inorganic, with metal halides being preferred. Possible metal halides for use in the process include halides of alkali metals, alkaline-earth metals, copper, silver, zinc, cadmium, mercury, aluminium, potassium, thallium, germanium, tin, lead, antimony, bismuth, titanium, zirconium, vanadium, niobium, tantalum, tellurium, chromium, molybdenum, tungsten, manganese, rhenium, iron, cobalt, nickel and rare-earth metals. Halides of alkali metals and alkaline-earth metals are particularly preferred. Chelating reagents for use in the process of JP-A-60-139 659 are described as being compounds having two or more coordinate groups in one ligand. One of the coordinate groups is preferably a tertiary amino group, a nitrogen-containing aromatic ring or an oxime. Oxidising agents are preferably molecular oxygen and/or organic nitro compounds, with molecular oxygen being especially preferred.

More recently, further processes for preparing carbamates have been proposed which, in spite of the drawbacks discussed in JP-A-60-139 654, employ a catalyst system comprising a halogen compound as a promotor or co-catalyst. Japanese patent application publication No. 63-297 356 (JP-A-63-297 356) discloses a process for the manufacture of carbamic acid esters, or carbamates, in which an amine or amino acid, carbon monoxide, a compound containing a hydroxy group and oxygen are reacted in the presence of a catalyst comprising a transition metal belonging to the platinum family of group VIII of the periodic system, or a salt thereof, a complex of a monovalent copper salt, trivalent titanium salt or vanadium salt and a basic compound. Palladium, platinum, rhenium and ruthenium are said to be preferred transition metals. Copper (I), titanium (III) and vanadium (III) as stated as being preferred for the metal complex. The Examples of JP-A-63-297 356 are limited to processes employing a catalyst comprising copper (I) chloride.

P. Giannoccaro (Journal of Organometallic Chemistry, 336 (1987), 271-278) has proposed a process for the preparation of N,N′-disubstituted ureas in which aromatic and aliphatic primary amines in alcohol

solution are reacted with carbon monoxide and oxygen under mild conditions in the presence of catalytic amounts of palladium (II) chloride or a palladium (II) complex. Under more drastic conditions of temperature and pressure carbamate esters are said to be obtained. The best yields are stated to have been obtained using a catalyst system comprising copper (II) chloride as a co-catalyst.

Surprisingly, it has been found that a palladium catalyst which comprises a ligand and which, contrary to the teaching of the prior art discussed above, is not reliant on the presence of a halide as a co-catalyst can be used to prepare certain carbamates in high yields.

Accordingly, the present invention provides a process for preparing carbamates having the general formula I:

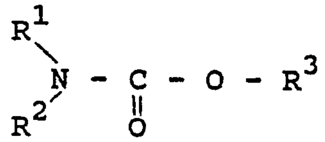

(I)

wherein $R^1$ represents a hydrogen atom or an alkyl group, $R^2$ represents an optionally substituted aryl group, and $R^3$ represents an alkyl, haloalkyl, alkenyl, haloalkenyl, alkynyl, haloalkynyl group or an optionally substituted aryl or heteroaryl group, which process comprises reacting an amine having the general formula II:

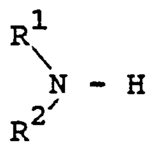

(II)

wherein $R^1$ and $R^2$ are as hereinbefore defined, or a symmetrical urea having the general formula III:

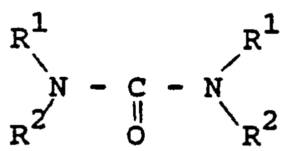

(III)

wherein $R^1$ and $R^2$ are as hereinbefore defined with a hydroxyl compound having the general formula IV:

$R^3$ - OH (IV)

wherein $R^3$ is as hereinbefore defined, carbon monoxide and oxygen in the presence of a catalyst comprising:

a) palladium;

b) an organo-nitrogen or organo-phosphorous ligand having a lone pair of electrons; and

c) a metal salt comprising a cation of a metal selected from the group consisting of copper, iron, vanadium, chromium, zinc, tin, uranium and cerium, and an anion of an acid having a pK of less than 2, except of a hydrohalogenic acid.

The starting materials for the process of the present invention may comprise an aromatic amine or a symmetrical aromatic urea or a combination of an amine and its corresponding symmetrical urea.

Alkyl and alkoxy groups preferably have from 1 to 12 carbon atoms, especially 1 to 6 carbon atoms. Alkenyl and alkynyl groups preferably have from 2 to 12 carbon atoms, especially 2 to 6 carbon atoms. Cycloalkyl groups preferably have from 3 to 10 carbon atoms, especially 3 to 8 carbon atoms. Alkyl, alkoxy, alkenyl and alkynyl groups may be straight chain or branched.

Optional substituents for aryl and heteroaryl groups may be independently selected from the group consisting of halogen atoms, especially fluorine, chlorine and bromine and alkyl, haloalkyl, alkoxy, alkoxycarbonylamino groups, and aryl, aryloxy, heteroaryl and heteroaryloxy groups themselves optionally substituted by one or more substituents independently selected from halogen atoms and alkyl, haloalkyl and alkoxy groups. Alkyl, haloalkyl and alkoxy groups when present as substituents preferably have from 1

to 4 carbon atoms especially 1 or 2 carbon atoms.

Preferably $R^1$ represents a hydrogen atom, ethyl or methyl, with a hydrogen atom being especially preferred.

$R^2$ referably represents an optionally substituted phenyl group. Most preferably, $R^2$ represents a phenyl group bearing substituents independently selected from the group consisting of halogen atoms, especially fluorine and chlorine, alkyl groups, especially methyl, haloalkyl groups, especially trifluoromethyl, heteroaryloxy groups, especially pyridyloxy, and phenoxy. Phenoxy and pyridyloxy preferably themselves bear one or more substituents selected from halogen atoms, especially chlorine, and haloalkyl groups, especially trifluoromethyl.

$R^3$ preferably represents an alkyl group, especially a $C_{1-4}$ alkyl group, most especially methyl, ethyl and 2-propyl, an alkynyl group, especially a $C_{2-5}$ alkynyl group, most especially 1-methylprop-2-ynyl, or a haloalkynyl group, especially a $C_{2-5}$ haloalkynyl group, most especially a halobutynyl group.

Carbamates of particular importance are those which are themselves active as pesticides, especially herbicides or insecticides, or are precursors to isocyanates which may, in turn, be used to prepare pesticidally active acyl ureas. Examples of carbamates of formula I which are active as either herbicides or insecticides include 4-chlorobut-2-ynyl 3-chlorophenylcarbamate, 1-methylprop-2-ynyl 3-chlorophenylcarbamate, isopropyl 3-chlorophenyl-carbamate, 3-ethoxycarbonylaminophenyl phenylcarbamate, and isopropyl phenylcarbamate. Examples of carbamates of formula I which may be used as precursors in the preparation of pesticidally active acyl ureas include methyl 4-chlorophenylcarbamate, methyl 2,4-difluoro-3,5-dichlorophenylcarbamate, methyl 2-fluoro-4-(2-chloro-4-trifluoro methylphenoxy)phenylcarbamate, methyl 3,5-dichloro-4-(3-chloro-5-trifluoromethyl-pyrid-2-yloxy)phenylcarbamate and methyl 4-trifluoromethylphenylcarbamate. Accordingly, the process of the present invention is particularly useful when the amine, and/or symmetrical urea, and hydroxyl compound are selected to produce such a compound.

The amines, symmetrical ureas, and hydroxyl compounds used as starting materials in the process according to the present invention are either commercially available compounds or can be readily obtained by standard preparative methods.

The process of the present invention is carried out in the presence of a catalyst comprising palladium. The palladium may be present in the form of palladium metal, preferably deposited on an inert carrier, for example alumina or carbon. Alternatively, the palladium may be present as palladium compounds, in particular complexes or salts of palladium. If the palladium is present as a palladium compound, the compound is preferably soluble in the reaction mixture.

Suitable palladium salts include palladium chloride, palladium bromide, palladium iodide and palladium carboxylates, for example palladium acetate, palladium propionate and palladium isobutyrate. The preferred salts are palladium carboxylates. Especially preferred is palladium acetate. Examples of suitable complexes of palladium include palladium acetylacetonate, sodium tetrachloropalladate, potassium tetrachloropalladate, and potassium tetraiodopalladate.

The quantity of palladium used in the process is not critical, but is conveniently in the range of 0.001% w/w to 10% w/w palladium metal and/or palladium compound, in particular in the range of 0.005% w/w to 3% w/w palladium metal and/or palladium compound, calculated on the amount of aromatic amine or symmetrical aromatic urea present.

The catalytic system further comprises an organo-nitrogen or organo-phosphorous ligand having a lone pair of electrons.

Suitable organo-phosphorous ligands which may be used in the process of the present invention include phosphites and tertiary phosphines of the general formula $P.OR^a.OR^b.OR^c$ or $PR^aR^bR^c$, wherein each of $R^a$, $R^b$ and $R^c$ independently represents an alkyl, cycloalkyl, aryl, alkaryl or aralkyl group each having up to 10 carbon atoms, optionally substituted by one or more substituents selected from fluorine and chlorine atoms, alkoxy and aryloxy groups each having up to 10 carbon atoms, in particular methoxy and phenoxy groups, cyano groups, and groups of formula $-PR^aR^b$; or $R^a$ and $R^b$ together with the interjacent heteroatom together represent a heteroaryl group. Preferred monodentate organophosphorus compounds include trimethylphosphine, triethylphosphine, tri-n-butylphosphine and triphenylphosphine. Examples of suitable bidentate phosphorus-containing ligands are 2-dimethyl-2-(methyldiphenylphosphino)-1,3-di-(diphenylphosphino)propane, tetramethyl diphosphinoethane, tetramethyl diphosphinopropane, tetraethyl diphosphinoethane, tetrabutyl diphosphinoethane, dimethyl diethyl diphosphinoethane, tetraphenyl diphosphinoethane, tetraperfluorophenyl diphosphinoethane, tetraphenyl diphosphinopropane, tetraphenyl diphosphinobutane, dimethyl diphenyl diphosphinopropane, tetratolyl diphosphinoethane, ditolyl diphenyl diphosphinoethane, tetratrifluoromethyl diphosphinotetrafluoroethane, tetraphenyl diphosphinoethene and derivatives thereof and 1,2-bis-(diphenylphosphino)benzene and derivatives thereof. Preferred are tetraphenyldiphosphinobutane, tetraphenyl diphosphinoethane and tetraphenyl diphosphinopropane.

Organo-nitrogen ligands which may conveniently be used in the process of the present invention include mono-, bi-, or poly-cyclic systems containing one or more nitrogen atoms. Systems containing 1 or 2 nitrogen atoms are preferred. Such cyclic organo-nitrogen compounds are preferably those having the general formula V or VI:

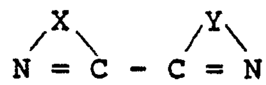

(V)

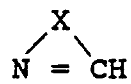

(VI)

wherein each of X and Y, which, in formula V, may be the same or different, represents a bridging group containing 3 or 4 atoms in the bridge of which at least 2 are carbon atoms. Any atoms in the bridging groups X and Y other than carbon atoms are preferably nitrogen atoms. In compounds having the general formula V, X and Y may be bound to each other by means of a connection in addition to that already formed by the carbon atoms shown in formula V. X and Y in formula V are preferably the same. The cyclic systems of formula V and VI are preferably aromatic. Preferred organo-nitrogen ligands are those having the general formula V.

Specific examples of suitable organo-nitrogen compounds include pyrrole, pyridine, pyrimidine, phenanthroline, pyrazine, benztriazole, 2,2′-dipyridyl, 2,2′-biquinoline, bis-pyridylketone, bis-pyridylglyoxal, and their alkyl-substituted derivatives. Analogous compounds containing other substituents, for example sulphonyl, carbonyl, alkoxy and halide moieties may also be used. The preferred compounds are 1,10-phenanthroline, 2,2′-dipyridyl, pyridine and alkyl-substituted pyridines such as the various picolines, for example alpha-picoline.

Preferred ligands are bidentate ligands, the nitrogen based ligands being especially preferred. The most preferred ligands are 1,10-phenanthroline and 2,2′-dipyridyl.

The ligand is generally present in a sufficient amount to give a molar ratio of ligand to palladium metal and/or palladium compound of from 0.5:1 to 30:1, preferably of from 5:1 to 20:1.

The catalytic system used in the process of the present invention further comprises a metal salt comprising a cation of a metal selected from the group consisting of copper, iron, vanadium, chromium, zinc, tin, uranium and cerium. The preferred metals are copper, iron, chromium and uranium. Most prefered are the cations of copper, especially the copper (II) cation. The metal salt further comprises an anion of an acid having a pK of less than 2, except the anion of hydrohalogenic acids. Preferred anions are those of sulphuric acid, fluoroboric acid, p-toluene sulphonic acid and alkyl substituted derivatives thereof, and perchloric acid. The most preferred anion is that of p-toluene sulphonic acid. Other anions that may also be used include those of benzene sulphonic acid, naphthalene sulphonic acid, 2,4,5-trichlorobenzene sulphonic acid, or corresponding bromo- and fluoro-analogues.

Examples of suitable metal salts include copper (I) tosylate, copper (II) tosylate, copper (II) chlorate, iron (II) chlorate, copper (II) fluoroborate, tin (IV) sulphate and uranium (IV) sulphate. The most preferred metal salt is copper (II) tosylate.

Carbamates are prepared by reacting an amine or a symmetrical urea with a hydroxyl compound, oxygen and carbon monoxide in the presence of the catalytic system described above.

Carbon monoxide and oxygen may be supplied by passing the gases into the reaction mixture during the period of the reaction, preferably throughout the period of the reaction. Oxygen may be supplied in the form of pure oxygen gas or an oxygen-containing stream, most conveniently as air. The oxygen and carbon monoxide streams may be supplied separately to the reaction mixture or, alternatively, may be combined. Typically a combined stream of carbon monoxide and air for supplying to the reaction mixture contains about 85% vol carbon monoxide and about 15% vol air.

The reaction may be performed in the presence of a solvent. Suitable solvents include any unreactive organic solvent, such as halogenated hydrocarbons, for example chloroform, 1,2-dichloroethane, chlorobenzene and the three dichlorobenzenes; hydrocarbons, for example hexane, cyclohexane, octane, benzene, toluene and the three xylenes; ethers, for example diethyl ether, tetrahydrofuran, dioxane and anisole; and esters such as ethyl acetate. Preferred solvents are xylene, chlorobenzene, and the three dichlorobenzenes. A particularly preferred solvent is 1,2-dichlorobenzene.

Alternatively, the hydroxyl compound may be present in the reaction mixture in excess and thereby act as a solvent.

The process is preferably effected at a temperature up to 175 °C, depending upon the operating pressure and the boiling point of the reagents. Preferred temperatures are in the range of from 70 °C to 160 °C, particularly 100 °C to 150 °C. The process may be carried out at atmospheric pressure except when the desired operating temperature exceeds the boiling temperature of the reaction mixture, in which case it will be necessary to employ elevated pressures. However, the process is preferably carried out at elevated pressures, for example in the range of from 5 bar to 150 bar, more preferably in the range of from 30 bar to 70 bar.

The present invention will be further illustrated by the following Examples. Products of the process were identified by high performance liquid chromatography (HPLC) and gas chromatography (GC) both via comparison with authentic reference samples.

## Preparation of catalyst

Copper (II) tosylate was prepared using the following procedure:

P-toluenesulphonic acid (20g) was added to water (500ml) and the resulting solution stirred whilst heating to 50°C. Powdered copper (II) hydroxide was slowly added to the solution during which the pH of the solution was monitored. Addition of the copper (II) hydroxide was stopped when the pH of the solution reached 7 (after the addition of about 10g of hydroxide). The resulting mixture was cooled to ambient temperature (20°C) and filtered. The solvent of the filtrate was evaporated leaving a solid residue. The residue was dried in a vacuum oven at 40°C to give green-blue crystals of copper (II) tosylate containing approximately 10% water in a yield of 21g.

The required catalyst was formed by combining commercially available palladium acetate, copper (II) tosylate and either 2,2′-dipyridyl or 1,10 phenanthroline in the quantities given in the following Examples.

## Example 1

### Preparation of methyl 4-chlorophenylcarbamate

Nitrogen was passed through a 150ml stainless steel reactor to render the atmosphere within the reactor inert. Methanol (70ml), p-chloroaniline (3.28g, 25mmol) and catalyst containing palladium acetate (0.06g, 0.25mmol), copper (II) tosylate (0.2g, 0.5mmol) and 1,10 phenanthroline (0.88g, 5mmol) were each added, under a nitrogen atmosphere, to the reactor. A mixture of 85% vol carbon monoxide and 15% vol air was passed into the reactor at a pressure of 51 bar and a flowrate of 2.5 Nl/h. The contents of the reactor were heated to 130 °C and the reaction allowed to proceed for 22.5 hours. After this time, the resulting mixture was analysed using HPLC and GC. The analysis showed a 100% conversion of the p-chloroaniline to methyl 4-chlorophenylcarbamate (4.6g, 25mmol) in a yield of 100%.

## Example 2

### Preparation of methyl 4-chlorophenylcarbamate

Nitrogen was passed through a 150ml stainless steel reactor to render the atmosphere within the reactor inert. Methanol (70ml), p-chloroaniline (3.28g, 25mmol) and catalyst containing palladium acetate (0.06g, 0.25mmol), copper (II) tosylate (0.2g, 0.5mmol) and 2,2′-dipyridyl (0.78g, 5mmol) were added, under a nitrogen atmosphere, to the reactor. A mixture of 85% vol carbon monoxide and 15% vol air was passed into the reactor at a pressure of 47 bar and a flowrate of 2.5Nl/h. The contents of the reactor were heated to 130 °C and the reaction allowed to proceed for 7 hours. After this time, the resulting mixture was analysed using HPLC and GC. The analysis showed a 70% conversion of the p-chloroaniline yielding methyl 4-chloro-phenylcarbamate (2.4g, 13mmol) in a yield of 52% and N,N′-bis(4-chlorophenyl)urea ( 1.0g, 3.6mmol).

Example 3

Preparation of methyl 4-chlorophenylcarbamate

The general procedure described in Example 2 was followed with the carbon monoxide/air mixture being passed into the reactor at a pressure of 6 bar. The reaction was allowed to proceed for 18 hours, after which time the resulting mixture was analysed using HPLC and GC. The analysis showed a 16% conversion of the p-chloroaniline yielding methyl 4-chlorophenylcarbamate (0.3g, 1.8mmol) in a yield of 7%.

Example 4

Preparation of methyl 2-fluoro-4-(2-chloro-4-trifluoro-methylphenoxy)phenyl carbamate

Nitrogen was passed through a 150ml stainless steel reactor to provide an inert atmosphere within the reactor. Methanol (70ml), 2-fluoro-4-(2-chloro-4-trifluoromethylphenoxy)aniline (4.74g, 17.5mmol) and catalyst containing palladium acetate (0.06g, 0.25mmol), copper (II) tosylate (0.20g, 0.5mmol) and 2,2'dipyridyl (0.78g, 5mmol) were each added, under a nitrogen atmosphere, to the reactor. A mixture of 85% vol carbon monoxide and 15% vol air was passed into the reactor at a pressure of 47 bar and a flowrate of 2.5Nl/h. The contents of the reactor were heated to 130°C and the reaction allowed to proceed for 20 hours. After this time, the contents of the reactor were analysed by HPLC and GC. The analysis showed a 98% conversion of 2-fluoro-4-(2-chloro-4-trifluoromethylphenoxy) aniline giving methyl 2-fluoro-4-(2-chloro-4-trifluoromethylphenoxy)phenyl carbamate (4.6g, 14.7mmol) in a yield of 84%.

**Claims**

1. A process for preparing carbamates having the general formula I:

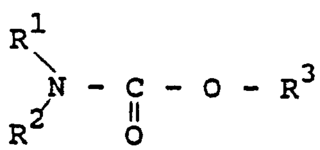

(I)

wherein $R^1$ represents a hydrogen atom or an alkyl group, $R^2$ represents an optionally substituted aryl group and $R^3$ represents an alkyl, haloalkyl, alkenyl, haloalkenyl, alkynyl, haloalkynyl group or an optionally substituted aryl or heteroaryl group, which process comprises reacting an amine having the general formula II:

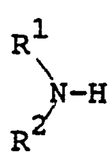

(II)

wherein $R^1$ and $R^2$ are as hereinbefore defined, or a symmetrical urea having the general formula III:

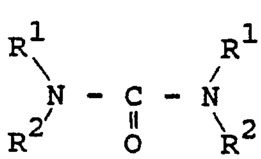

(III)

wherein $R^1$ and $R^2$ are as hereinbefore defined, carbon monoxide and oxygen in the presence of a catalyst comprising:

a) palladium;

b) an organo-nitrogen or organo-phosphorous ligand having a lone pair of electrons; and

c) a metal salt comprising a cation of a metal selected from the group consisting of copper, iron, vanadium, chromium, zinc, tin, uranium and cerium, and an anion of an acid having a pK of less than 2, except of a hydrohalogenic acid.

2. A process according to claim 1, wherein the catalyst comprises an organo-nitrogen ligand having the general formula V:

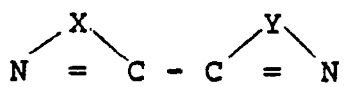 (V)

wherein X and Y represent the same or different bridging groups each of which has 3 or 4 atoms in the bridge, of which atoms at least two are carbon atoms and which groups X and Y may be bound to each other by means of a connection in addition to that already formed by the carbon atoms shown in formula V.

3. A process according to claim 1 or 2, wherein the catalyst comprises a salt or complex of palladium.

4. A process according to any one of claims 1 to 3, wherein the metal salt comprises a cation of copper.

5. A process according to any preceding claim, wherein the metal salt comprises an anion of the group consisting of sulphate, hydrocarbylsulphonates, fluoroborate and perchlorate.

6. A process according to claim 5, wherein the anion is tosylate.

7. A process according to any preceding claim, wherein the pressure is in the range of from 5 to 150 bar.

8. A process according to claim 7, wherein the pressure is in the range of from 30 to 70 bar.

9. A process according to any preceding claim, wherein the temperature is in the range of from 70 °C to 160 °C.

10. A process according to any preceding claim, wherein the carbon monoxide is passed into the reaction mixture continuously during the period of the reaction.

European Patent Office

# EUROPEAN SEARCH REPORT

Application Number

EP 90 20 0960

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | EP-A-0 235 865 (SHELL)<br>* claims 3,4,5 *<br>--- | 1 | C 07 C 269/04<br>C 07 C 271/30 |
| Y | EP-A-0 231 045 (SHELL)<br>* claim 1 *<br>--- | 2 | |
| Y | EP-A-0 173 457 (BP)<br>* claim 6 *<br>--- | 1,2 | |
| A | EP-A-0 200 556 (BP)<br>* claims 6,10 *<br>--- | 1,4 | |
| A | EP-A-0 195 515 (DUPONT)<br>* page 6, lines 22-34; page 7, lines 1,2; claim 1 *<br>--- | 1,4 | |
| A | EP-A-0 086 281 (SHELL)<br>* claims 1,4,11 *<br>----- | 1,6 | |
| | | | TECHNICAL FIELDS SEARCHED (Int. Cl.5) |
| | | | C 07 C 269/04<br>C 07 C 271/30 |

The present search report has been drawn up for all claims

| Place of search | Date of completion of the search | Examiner |
|---|---|---|
| BERLIN | 16-07-1990 | KAPTEYN H G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)